# EUROPÄISCHE PATENTANMELDUNG

(11) **EP 1 211 308 A1**
(43) Veröffentlichungstag der Anmeldung: **05.06.2002**
(21) Anmeldenummer: 01127196.2
(22) Anmeldetag: 16.11.2001
(51) Int. Cl.: C12M 1/107, B09B 3/00

(54) **Verfahren zur Verwertung von Tiermehl**

(30) Priorität: 01.12.2000 DE 10060071; 04.01.2001 DE 10100174
(71) Anmelder: EnviTec-Mall - Umweltsysteme GmbH & Co. KG, 48369 Saerbeck (DE)
(72) Erfinder: Tenbrink, Jürgen, 48565 Steinfurt (DE)
(74) Vertreter: Patentanwälte Lippert, Stachow, Schmidt & Partner

(57) **Zusammenfassung**

Die Erfindung betrifft ein Verfahren zur Teilverwertung von Tiermehl, welches folgende Verfahrensschritte umfasst:
- Anmaischen des Tiermehls mit Wasser
- anaerobe Fermentation der Maische in einem im Wesentlichen abgeschlossenen Gärbehälter,
- Abführen und thermische Verwertung von bei der Fermentation angefallenem Gas,
- Feststoffseparation des bei der Fermentation anfallenden Gärrests und
- Aufbereitung der bei der Separation angefallenen Flüssigkeit bis zur hygienischen Unbedenklichkeit.

## Beschreibung

Die Erfindung betrifft ein Verfahren zur Teilverwertung von Tiermehl und/oder dessen Vorprodukten.

Grundsätzlich wäre die Verwertung von Tiermehl durch Verbrennung in bekannter Art und Weise möglich. Eine solche Verbrennung ist jedoch mit erheblichen Kosten verbunden, zumal die Verbrennung bei erhöhten Temperaturen durchgeführt werden muss um sicherzustellen, dass die in dem Tiermehl etwa enthaltenen Prionen und Eiweiße zerstört werden.

Der Erfindung liegt daher die Aufgabe zugrunde, ein preiswertes Verfahren zur Verwertung von Tiermehl bereitzustellen, bei dem zumindest die Menge einer Verbrennung zuzuführender Substanz reduziert wird.

Die Aufgabe wird gelöst durch ein Verfahren zur Teilverwertung von Tiermehl, folgende Verfahrensschritte umfassend:
- Anmaischen des Tiermehls und/oder eines Tiermehl-Vorprodukts mit Wasser,
- anaerobe Fermentation der Maische in einem im Wesentlichen abgeschlossenen Gärbehälter,
- Abführen und thermische Verwertung von bei der Fermentation anfallendem Gas,
- Feststoffseparation des bei der Fermentation anfallenden Gärrests und
- Aufbereitung der bei der Separation anfallenden Flüssigkeit.

Der Erfindung liegt der Gedanke zugrunde, das Tiermehl erst einer Teilverwertung zuzuführen, bei der der Anteil der organischen Substanz zunächst durch Fermentation bzw. Vergärung unter Gewinnung von Biogas reduziert wird. Mit dem erfindungsgemäß vorgeschlagenen Verfahren ist es möglich, ca. 60 % der organischen Substanz biologisch anaerob abzubauen, sodass nur noch 40 % der Ausgangssubstanz einer Verbrennung zugeführt werden müssen, was ein beachtliche Kosteneinsparung nach sich zieht.

Das Verfahren geht davon aus, dass die verbleibenden Feststoffe durch Verbrennung entsorgt werden, wobei die Entsorgungskosten durch die Massereduktion beim biologischen Abbau herabgesetzt werden. Aus dem Prozess entstehendes Abwasser wird soweit aufbereitet, dass ein hygienisch unbedenklicher Abfluss entsteht, der abgeleitet werden kann.

Vorzugsweise erfolgt die Aufbereitung der bei der Separation anfallenden Flüssigkeit derart, dass wenigstens durch vorzugsweise mehrstufige Filtration wenigstens Kolloide und Eiweiße einschließlich Prionen aus der Flüssigkeit entfernt werden.

Bei einer besonders bevorzugten Variante des Verfahrens umfasst die Aufbereitung der Flüssigkeit eine der mehrstufigen Filtration nachgeschaltete Umkehrosmose. Die Anwendung einer solchen Umkehrosmose hat sich als besonders vorteilhaft herausgestellt, da der Abscheidungsgrad bei der Umkehrosmose besonders präzise und auf feinste gelöste Bestandteile einer Flüssigkeit bis hin zu Molekülgröße einstellbar ist.

Zweckmäßigerweise wird die Filtration als mehrstufige Membranfiltration bzw. Nanofiltration durchgeführt, die der Umkehrosmose vorgeschaltet ist.

Wenigstens ein Teil der bei der thermischen Verwertung des Gases, das hauptsächlich aus CH₄, CO₂, H₂S und Wasserdampf besteht, anfallenden Wärme kann zur Beheizung des Gärbehälters verwendet werden.

Ebenso kann ein Teil der bei der thermischen Verwertung des Gases anfallenden Wärme zur Trocknung des bei der Separation anfallenden Feststoffs verwendet werden.

Vorzugsweise wird der Filterrückstand aus der Filtration dem Gärrest vor der Separation wieder zugeführt.

Das aus dem Gas anfallende Kondensat kann der Flüssigkeit nach der Feststoffseparation zugeführt werden.

Wenigstens ein Teil des Feststoffaustrags aus der Umkehrosmose kann dem Feststoffaustrags aus der Separation zugeführt werden, auch kann dieser Feststoffaustrags teilweise dem Gärrest aus der Fermentation vor der Separation zugeführt werden.

Zweckmäßigerweise werden die anfallenden Feststoffe, gegebenenfalls nach einer Trocknung, einer thermischen Verwertung zugeführt. Wie vorstehend bereits erwähnt, kann zur Trocknung die aus der thermischen Verwertung des Gases anfallende Wärme herangezogen werden.

Bei der bevorzugten Variante des erfindungsgemäßen Verfahrens wird das bei der Fermentation anfallende Gas einer Einrichtung zur Strom- und Wärmeerzeugung mittels Kraft-Wärmekopplung zugeführt. Solche Einrichtungen zur Kraft-Wärmekopplung sind allgemein bekannt.

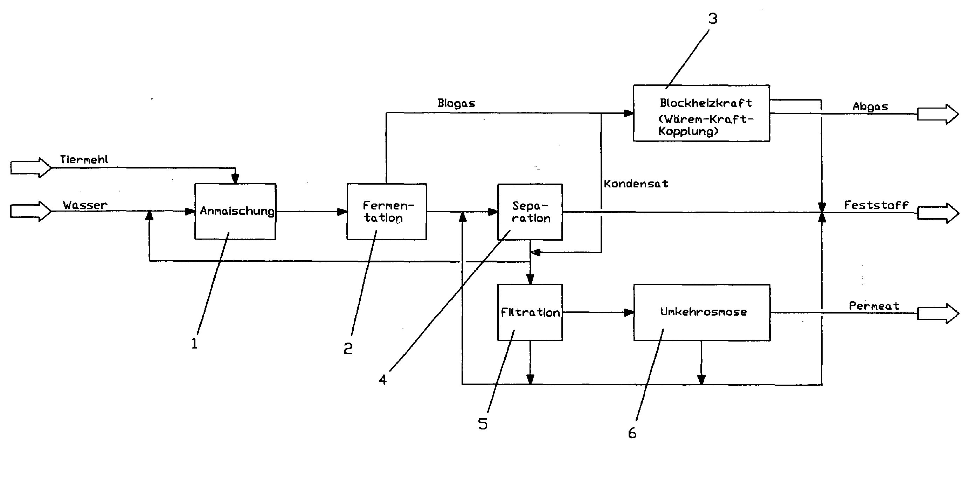

Die Erfindung wird nachstehend anhand eines in der Zeichnung dargestellten Ausführungsbeispiels erläutert.

Wie dem Fließbild zu entnehmen ist, wird Tiermehl unter Zugabe von Wasser in einem Maischebehälter 1 angemaischt und einem Fermenter 2 zugeführt. Das Tiermehl hat in der Regel einen Feuchtigkeitsgehalt von ca. 15 %. In dem Maischebehälter 1 wird der Trockensubstanzgehalt durch Zugabe von Wasser auf ≤ 12 % eingestellt. In dem Fermenter 2 erfolgt eine anaerobe Vergärung unter Bildung von Biogas, d.h. CH₄, CO₂, H₂S, NH₃ und Wasserdampf, welches einem mit 3 bezeichneten Blockheizkraftwerk zugeführt wird. Das Blockheizkraftwerk 3 ist als Einrichtung zur Kraft-Wärmekopplung ausgebildet, in dem Verbrennungskraftmaschinen mit dem Biogas und der Ausnutzung der von den Verbrennungskraftmaschinen erzeugten mechanischen und thermischen Energie betrieben werden.

Nach Abbau organischer Substanz in dem Fermenter 2 zu Biogas wird aus diesem der Gärrest mit einem Wasseranteil von ca. 90 bis 95 % abgezogen und einem Separator 4 zur Trennung von Feststoff und Flüssigkeit zugeführt. Als Separator kommen Schneckenpressen, Siebbandpressen, Membranfilterpressen, Kammerfilterpressen, Dekanter oder ähnliche Apparaturen in Betracht.

Die aus dem Separator 4 abgezogene Flüssigkeit wird wiederum zur Anmaischung des dem Maischebehälter 1 zugeführten Tiermehls verwendet. Durch die Zugabe von Flüssigkeit aus der Separation wird die dem Fermenter zugeführte organische Substanz in Form von Tiermehl mit den Organismen für die Fermentation angeimpft. Der Fermenter 2 ist vorzugsweise beheizt.

Wie bereits vorstehend erwähnt, wird das aus dem Fermenter 2 abgezogene Biogas in dem mit 5 bezeichneten Blockheizkraftwerk beispielsweise in einem nicht dargestellten Gasmotor verbrannt und auf bekannte Art und Weise mit Hilfe eines Generators zu Strom umgewandelt. Die bei dem Verbrennungsprozess entstehende Wärme kann zumindest teilweise zur Beheizung des Fermenters 2 verwendet werden. Bei der Verbrennung werden alle patogenen Keime abgetötet.

Der aus dem ein- oder mehrstufig ausgebildeten Separator 4 abezogene Feststoff wird schließlich ebenso einer Verbrennung zugeführt. Um den Trockensubstanzgehalt zu erhöhen, können die festen Bestandteile zusätzlich mit der Wärme aus dem Blockheizkraftwerk 3 getrocknet werden.

Das bei der Abführung des Gases aus dem Fermenter 2 in der Regel in der Gasleitung anfallende Kondensat wird der aus dem Separator 4 abgezogenen Flüssigkeit zugegeben. Die flüssige Phase aus der Fest-Flüssigtrennung im Separator 4 ist organisch belastet und hygienisch nicht unbedenklich. Wie vorstehend bereits erwähnt, wird ein Teil dieser Flüssigkeit zur Impfung des Substrats verwendet. Der Rest der in dem Separator 4 abgetrennten flüssigen Phase wird zuerst einer mehrstufigen Filtration 5 zugeführt. Diese Filtration 5 wird als Membranfiltration durchgeführt. Der Filterdurchgang wird einer nachgeschalteten Umkehrosmose 6 zugeführt. Der Filterrückstand sowie das in der Umkehrosmose 6 zurückgehaltene hochkonzentrierte Material werden teilweise wieder den Gärresten vor dem Separator 4 zugeführt. Ein Teil dieses Feststoffs kann auch dem aus dem Separator 4 abgezogenen Feststoff, der zur Verbrennung vorgesehen ist, zugeführt werden. Dieser Feststoff hat vor einer etwaigen Trocknung einen Feuchtigkeitsgehalt von ca. 30 %.

Das aus der Umkehrosmose 6 erhaltene Permeat schließlich ist hygienisch unbedenklich und kann abgeleitet werden.

Das Verhältnis desjenigen Feststoffs aus der Umkehrosmose 6, der dem Gärrest vor dem Separator 4 zugeführt wird, zu demjenigen Feststoff, der dem Feststoff hinter dem Separator 4 zugeführt wird, ist einstellbar.

Bei einem Einsatz von ca. 50.000 Tonnen Tiermehl werden ca. 30.000 Tonnen Gas erhalten, was eine beachtliche Ausbeute darstellt. Hierbei werden ca. 60 % der organischen Substanz bzw. des Substrats bei der Fermentation abgebaut.

Nur der Vollständigkeit halber sei erwähnt, dass der Porendurchmesser der bei der Umkehrosmose verwendeten Membrane ≤ 1 µm beträgt.

Die Ausbildung des Fermenters 2 sowie auch des Maischebehälters 1, der auch mit üblichen Dosier- und Rühreinrichtungen versehen ist, ist dem Fachmann bekannt.

Anstelle der in dem Ausführungsbeispiel vorgeschlagenen Umkehrosmose können auch andere Behandlungsschritte eingesetzt werden, mit deren Hilfe der Filterdurchgang aus der Filtratioin 5 hygienisiert werden kann. Hier kommen unter anderem oxidative, chemische oder thermische Verfahren in Betracht.

### Bezugzeichenliste

- 1: Maischebehälter
- 2: Fermenter
- 3: Blockheizkraftwerk
- 4: Separator
- 5: Filtration
- 6: Umkehrosmose

## Patentansprüche

1. Verfahren zur Teilverwertung von Tiermehl und/oder dessen Vorprodukten, folgende Verfahrensschritte umfassend:
- Anmaischen des Tiermehls mit Wasser
- anaerobe Fermentation der Maische in einem im Wesentlichen abgeschlossenen Gärbehälter,
- Abführen und thermische Verwertung von bei der Fermentation anfallendem Gas,
- Feststoffseparation des bei der Fermentation anfallenden Gärrests und
- Aufbereitung der bei der Separation anfallenden Flüssigkeit bis zur hygienischen Unbedenklichkeit.

2. Verfahren nach Anspruch 1, **dadurch gekennzeichnet, dass** die Aufbereitung der bei der Separation anfallenden Flüssigkeit derart erfolgt, dass wenigstens durch vorzugsweise mehrstufige Filtration wenigstens Kolloide und Eiweiße einschließlich Prionen aus der Flüssigkeit entfernt werden.

3. Verfahren nach Anspruch 1, **dadurch gekennzeichnet, dass** die Aufbereitung der Flüssigkeit eine der mehrstufigen Filtration nachgeschaltete Umkehrosmose umfasst.

4. Verfahren nach Anspruch 1 oder 2, **dadurch gekennzeichnet, dass** die Filtration als Membranfiltration durchgeführt wird.

5. Verfahren nach einem der Ansprüche 1 bis 4, **dadurch gekennzeichnet, dass** wenigstens ein Teil der bei der thermischen Verwertung des Gases anfallenden Wärme zur Beheizung des Gärbehälters verwendet wird.

6. Verfahren nach einem der Ansprüche 1 bis 5, **dadurch gekennzeichnet, dass** ein Teil der bei der thermischen Verwertung des Gases anfallenden Wärme zur Trockung des bei der Separation anfallenden Feststoffs verwendet wird.

7. Verfahren nach einem der Ansprüche 1 bis 6, **dadurch gekennzeichnet, dass** der Filterrückstand aus der Filtration dem Gärrest vor der Separation zugeführt wird.

8. Verfahren nach einem der Ansprüche 1 bis 7, **dadurch gekennzeichnet, dass** das aus dem Gas anfallende Kondensat der Flüssigkeit nach der Feststoffseparation zugeführt wird.

9. Verfahren nach einem der Ansprüche 1 bis 8, **dadurch gekennzeichnet, dass** wenigstens ein Teil des Feststoffaustrags aus der Umkehrosmose dem Feststoffaustrag aus der Separation zugeführt wird.

10. Verfahren nach einem der Ansprüche 1 bis 9, **dadurch gekennzeichnet, dass** wenigstens ein Teil des Feststoffaustrags aus der Umkehrosmose dem Gärrest vor der Separation zugeführt wird.

11. Verfahren nach einem der Ansprüche 1 bis 10, **dadurch gekennzeichnet, dass** die anfallenden Feststoffe gegebenenfalls nach einer Trocknung einer thermischen Verwertung zugeführt werden.

12. Verfahren nach einem der Ansprüche 1 bis 11, **dadurch gekennzeichnet, dass** das bei der Fermentation anfallende Gas einer Einrichtung zur Stromund Wärmeerzeugung mittels Kraft-Wärmekopplung zugeführt wird.
